# EUROPEAN PATENT APPLICATION

(11) **EP 3 945 528 A1**
(43) Date of publication of application: **02.02.2022**
(21) Application number: 21188253.5
(22) Date of filing: 28.07.2021
(51) Int. Cl.: G16H 20/30, G16H 70/20, A61B 5/00, A63B 24/00, G09B 19/00, G16H 40/20

(54) **TRAINING SYSTEM, TRAINING METHOD, AND PROGRAM**

(30) Priority: 28.07.2020 JP 2020127545
(71) Applicant: TOYOTA JIDOSHA KABUSHIKI KAISHA, Toyota-shi, Aichi-ken 471-8571 (JP)
(72) Inventor: YUASA, Hisataka, Aichi-ken, 471-8571 (JP); MIYAGAWA, Toru, Aichi-ken, 471-8571 (JP); NAKASHIMA, Issei, Aichi-ken, 471-8571 (JP); KOBAYASHI, Makoto, Aichi-ken, 471-8571 (JP); IWATA, Takuya, Aichi-ken, 471-8571 (JP); SUGA, Keisuke, Aichi-ken, 471-8571 (JP); IMAIDA, Masayuki, Aichi-ken, 471-8571 (JP); SAITOH, Eiichi, Aichi-ken, 471-8571 (JP); OTAKA, Yohei, Aichi-ken, 471-8571 (JP); HIRANO, Satoshi, Aichi-ken, 471-8571 (JP)
(74) Representative: D Young & Co LLP

(57) **Abstract**

A training system (10) generates a training plan for enabling a trainee to perform a plurality of kinds of training, and includes a storage unit (11), a track record information accepting unit (12), and a plan generation unit (13). The storage unit (11) stores training information including the content of training, therapist information indicating the type of a therapist who is in charge of the training and assists the trainee, and statistical information regarding the function recovery in the training performed by a plurality of other trainees. The track record information accepting unit (12) accepts track record information regarding the physical action of the trainee. The plan generation unit (13) generates a training plan including the content of the training, the type of the therapist, and an execution period of the training based on the training information, the therapist information, and the statistical information and outputs the generated training plan.

## Description

### BACKGROUND

The present disclosure relates to a training system, a training method, and a program.

Training systems for the purpose of recovering functions of physical actions of trainees have been developed. For example, a walking training apparatus according to Japanese Unexamined Patent Application Publication No. 2015-223294 includes a walking assist device, a first tensile means, and a second tensile means. The walking assist device, which is mounted on a leg part of a trainee, assists walking of the trainee. The first tensile means pulls at least one of the walking assist device and the leg part of the trainee upward and frontward.
The second tensile means pulls at least one of the walking assist device and the leg part of the trainee upward and rearward.

### SUMMARY

The aforementioned training system is aimed at giving training in a specific action, namely, flat ground walking, of trainee's physical actions. However, there are a number of training exercises in accordance with physical actions for the purpose of recovering functions. In order for a trainee to recover his/her physical actions efficiently, it is desired to carry out training plans based on objective information.

The present disclosure has been made in order to solve the above problem, and aims to provide a training system and the like that enable a trainee to perform function recovery training efficiently.

A training system according to the present disclose generates a training plan for enabling a trainee to perform a plurality of kinds of training for the purpose of recovering functions of a physical action of the trainee, and includes a storage unit, a track record information accepting unit, and a plan generation unit. The storage unit stores training information including the content of the plurality of kinds of training, therapist information indicating the type of a therapist who is in charge of the training and assists the trainee, and statistical information regarding the function recovery in the training performed by a plurality of other trainees.

The track record information accepting unit accepts track record information regarding the physical action of the trainee. The plan generation unit generates, when it has accepted the track record information, a training plan including the content of the training that the trainee conducts, the type of the therapist, and an execution period of the training based on the training information, the therapist information, and the statistical information and outputs the generated training plan.

According to the above structure, the training system is able to output a training plan in accordance with objective information.

In the aforementioned training system, the storage unit may store, as the statistical information, information in which the content of the training performed by the plurality of other trainees, types of the therapists who have been in charge of the training, and degrees of recovery of physical actions of the plurality of other trainees are associated with one another. Accordingly, the training system is able to generate a training plan using the above statistical information.

In the aforementioned training system, the track record information accepting unit may further accept profile information of the trainee. Accordingly, the training system is able to generate a training plan in accordance with the profile of the trainee.

In the aforementioned training system, the track record information accepting unit may accept information regarding an action level of the trainee set using at least information regarding the type of a tool that the trainee uses in the physical action and the level of achievement of the physical action, and the plan generation unit may generate the training plan based on the action level. Accordingly, the training system is able to present a training plan in an aspect in which the user can easily understand the training plan using the action level.

In the aforementioned training system, the track record information accepting unit may further accept a target of the training, and the plan generation unit may generate the training plan based on the target. Accordingly, the training system is able to generate a training plan that may meet the accepted target.

In the aforementioned training system, the storage unit may further store identification information on the therapist as the therapist information, and the plan generation unit may generate, when it generates the training plan after a predetermined timing, the training plan in accordance with the length of an execution period of the training determined in accordance with a combination of the trainee with the therapist based on a combination of the trainee, the training that the trainee has performed before the predetermined timing, and the identification information on the therapist who has been in charge. Accordingly, the training system is able to generate a training plan in which a combination of a specific trainee with a specific therapist is taken into account.

In the aforementioned training system, the plan generation unit may set, in the combination, the priority of a first therapist who takes care of the trainee for a first period to be higher than the priority of a second therapist who takes care of the trainee for a second period shorter than the first period, and generates the training plan based on the set priority. Accordingly, the training system is able to generate a training plan in which a specific therapist preferentially assists a specific trainee.

In the aforementioned training system, the plan generation unit may generate, when it generates the training plan for a plurality of trainees, the training plan by restricting the number of training exercises that the therapist having one identifier is in charge during the same time based on the identification information. Accordingly, the training system is able to generate a training plan in a simple way and efficiently.

In the aforementioned training system, the storage unit may further store constraint information regarding constraint conditions at a time of training that the trainee or the therapist performs in a preset period, and the plan generation unit may generate the training plan based on the constraint conditions. The training system is able to generate a training plan in a flexible way and efficiently in view of the above constraint conditions.

In the aforementioned training system, the plan generation unit may generate, when it generates the training plan including a plurality of training exercises for one trainee, the training plan including an order in which the plurality of training exercises are performed or time when the plurality of training exercises are started. Accordingly, the training system is able to generate a more efficient training plan.

In the aforementioned training system, the plan generation unit may generate a plurality of different training plans that can be executed and output the generated plans. Accordingly, the training system is able to present a plurality of training plans that can be selected to the user.

In the aforementioned training system, the track record information accepting unit may accept information from a sensor that has detected the physical action of the trainee as the track record information. Further, in the aforementioned training system, the track record information accepting unit may accept image information from a camera that has captured images of the physical action of the trainee as the track record information. Accordingly, the training system is able to easily acquire the track record information.

A training method according to the present disclosure is a method for generating a training plan for enabling a trainee to perform a plurality of kinds of training for the purpose of recovering functions of a physical action of the trainee, and includes a storing step, a track record information accepting step, and a plan generation step. The storing step stores training information including the content of the plurality of kinds of training, therapist information indicating the type of a therapist who is in charge of the training and assists the trainee, and statistical information regarding the function recovery in the training performed by a plurality of other trainees. The track record information accepting step accepts track record information regarding the physical action of the trainee. The plan generation step generates, when the track record information has been accepted, a training plan including the content of the training that the trainee conducts, the type of the therapist, and an execution period of the training based on the training information, the therapist information, and the statistical information and outputs the generated training plan.

According to the above structure, the training method is able to output a training plan in accordance with objective information.

A program for causing a computer to execute a training method for generating a training plan for enabling a trainee to perform a plurality of kinds of training for the purpose of recovering functions of a physical action of the trainee is provided, and the training method includes a storing step, a track record information accepting step, and a plan generation step. The storing step stores training information including the content of the plurality of kinds of training, therapist information indicating the type of a therapist who is in charge of the training and assists the trainee, and statistical information regarding the function recovery in the training performed by a plurality of other trainees. The track record information accepting step accepts track record information regarding the physical action of the trainee. The plan generation step generates, when the track record information has been accepted, a training plan including the content of the training that the trainee conducts, the type of the therapist, and an execution period of the training based on the training information, the therapist information, and the statistical information and outputs the generated training plan.

According to the above structure, the training method is able to output a training plan in accordance with objective information.

According to the present disclosure, it is possible to provide a training system and the like that enable a trainee to perform function recovery training efficiently.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a block diagram of a training system according to a first embodiment;
Fig. 2 is a flowchart of the training system according to the first embodiment;
Fig. 3 is a diagram showing training information and therapist information according to the first embodiment;
Fig. 4 is a diagram showing an action level according to the first embodiment;
Fig. 5 is a diagram showing statistical information according to the first embodiment;
Fig. 6 is a diagram showing track record information of the training system according to the first embodiment;
Fig. 7 is a first diagram showing a training plan according to the first embodiment;
Fig. 8 is a second diagram showing the training plan according to the first embodiment;
Fig. 9 is a block diagram of a training system according to a second embodiment;
Fig. 10 is a flowchart of the training system according to the second embodiment;
Fig. 11 is a diagram showing training information according to the second embodiment;
Fig. 12 is a first diagram showing a training plan according to the second embodiment; and
Fig. 13 is a second diagram showing the training plan according to the second embodiment.

### DESCRIPTION OF EMBODIMENTS

Hereinafter, the present disclosure will be explained with reference to embodiments of the present disclosure. However, the disclosure set forth in the claims is not limited to the following embodiments. Further, not all the structures explained in the embodiments may be necessary as means for solving the problem. For a purpose of clarifying the description, the following description and the drawings will be omitted and simplified as appropriate. Throughout the drawings, the same components are denoted by the same reference symbols and overlapping descriptions will be omitted as necessary.

### <Embodiments>

Hereinafter, with reference to the drawings, embodiments of the present disclosure will be described. A training system according to the embodiments generates a training plan for enabling a trainee to carry out a plurality of kinds of training for the purpose of recovering functions of his/her impaired physical actions. The trainee here is, for example, a person suffering from paralysis in a part of his/her body, which is caused by a stroke (cerebrovascular disease) or a spinal cord injury. The trainee's physical action functions have been impaired due to the above disease. In this embodiment, physical actions are, for example, actions included in Activities of Daily Living (ADL) and include actions such as eating, toileting, dressing, walking, etc.

A trainee carries out a plurality of kinds of training in order to recover the functions of his/her impaired physical actions. To be specific, for example, training for recovering functions of a physical action eating includes training of a meal intake action, training of a swallowing action, and the like. Further, the training for recovering functions of the toileting action includes, for example, training of a moving action for moving to each of a bed, a wheelchair, and a toilet seat, training for toileting, and the like. Further, the training for recovering the function of the walking action includes, for example, training for maintaining the sitting posture, training for maintaining the standing posture, training of flat ground walking, training of walking on a slope, and the like. Further, the training includes, besides the training in which the trainee actively carries out physical actions as described above, passive training such as a massage in which the trainee receives stimulation to his/her joints or muscles from an assistant. The aforementioned training is also referred to as rehabilitation.

Some training performed using the training system may require assistance by therapists. Therapists are persons who have expertise that is necessary to assist trainees. In this embodiment, the therapists include a physical therapist, an occupational therapist, and a speech therapist. The therapists assist training performed by the trainees in accordance with expertise that they each have.

The physical therapist is a person who offers a physical therapy for persons who have physical disabilities mainly for the purpose of recovering fundamental action abilities. The physical therapy is a therapeutic treatment performed on persons whose functions of physical actions have been impaired due to diseases, injuries, disabilities and the like for the purpose of maintaining or improving these functions. The physical therapist carries out a physical therapy by using exercises, heat, electricity and the like. The physical therapist is also referred to as a Physical Therapist (PT).

The occupational therapist is a person who offers an occupational therapy for persons who have physical or mental disabilities mainly for the purpose of recovering their applicative action abilities or social adaptability. The "occupation" here includes daily activities such as bathing, dressing, and toileting. The occupational therapist is also referred to as OT.

The speech therapist is a person who carries out language training and other training exercises and provides tests, advice, guidance, and other assistance necessary for the above training exercises for persons who have disabilities in their speech functions, language functions, or hearing for the purpose of maintaining and improving these functions. The speech therapist further conducts training such as meal intake or swallowing under the instructions of doctors or dentists. The speech therapist is also referred to as a speech-language-hearing therapist or ST.

### <First Embodiment>

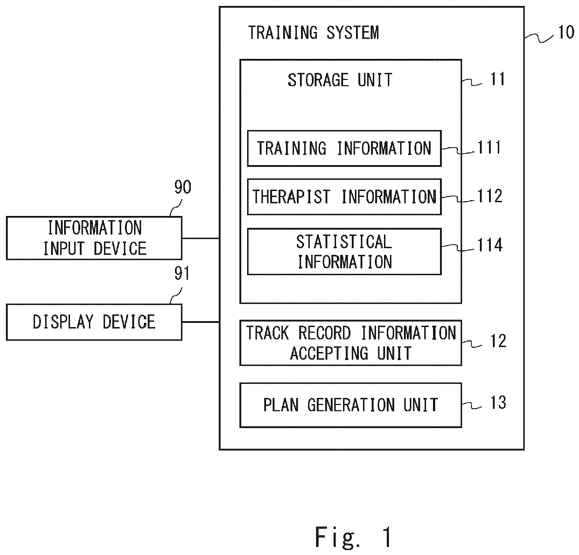

Hereinafter, a first embodiment will be described. Fig. 1 is a block diagram of a training system according to the first embodiment. A training system 10 shown in Fig. 1 includes, for example, a flash memory, a circuit board on which a Dynamic Random Access Memory (DRAM), a Central Processing Unit (CPU) and the like are mounted, and executes a control program stored in the memory, thereby implementing the function of the system. The training system 10 mainly includes a storage unit 11, a track record information accepting unit 12, and a plan generation unit 13. Further, these components included in the training system 10 are connected to one another as appropriate in such a way that they can communicate with one another. The training system 10 is further connected to an information input device 90 and a display device 91 in such a way that they can communicate with each other.

Hereinafter, main components of the training system 10 will be described.

The storage unit 11 includes a non-volatile memory such as a flash memory, an Erasable Programmable Read Only Memory (EPROM), or a Solid State Drive (SSD). The storage unit 11 stores training information 111, therapist information 112, and statistical information 114. The training information 111 includes the content of a plurality of kinds of training. The therapist information 112 is information indicating the type of a therapist who is in charge of training and assists a trainee. The statistical information 114 is information regarding the function recovery in training exercises that a plurality of other trainees have performed. When the training plan is generated, the storage unit 11 stores the aforementioned information in advance. Then the storage unit 11 supplies the aforementioned information to the plan generation unit 13 as appropriate.

The track record information accepting unit 12 is an interface that accepts information supplied from the information input device 90. The track record information accepting unit 12 accepts the track record information regarding the physical actions of the trainee. The track record information includes information regarding the physical actions of a trainee for whom a training plan is to be generated and includes, for example, information indicating which kind of action the trainee may perform using which tool. The track record information may be indicated by a preset index. The track record information accepting unit 12 supplies the accepted track record information to the plan generation unit 13.

The plan generation unit 13 generates, when it has accepted the track record information, the training plan in accordance with the accepted track record information and outputs the generated training plan. The training plan includes, for example, the content of the training performed by the trainee, the type of a therapist who is in charge of this training, and an execution period of the training. The plan generation unit 13 generates this training plan by using the training information 111, the therapist information 112, and the statistical information 114. The plan generation unit 13 outputs the generated training plan to the display device 91.

Next, a structure connected to the training system 10 will be described. The information input device 90 includes, for example, a character input function such as a keyboard. The information input device 90 accepts a predetermined operation by the user and inputs, for example, various kinds of information such as track record information to the training system 10 in accordance with the accepted operation. The display device 91, which includes, for example, a liquid crystal panel or an organic Electronic Luminescent (EL) panel, is able to present various kinds of information to the user. The display device 91 receives information regarding the training plan from the plan generation unit 13 and displays the received information in an aspect in which the user can visually recognize the received information.

Note that the information input device 90 and the display device 91 may be configured integrally with the training system 10. Further, the information input device 90 and the display device 91 may each be a device including an information input/output function, such as a smartphone or a tablet that is separate from the training system 10, and may be connected to the training system 10 in such a way that they can communicate with each other.

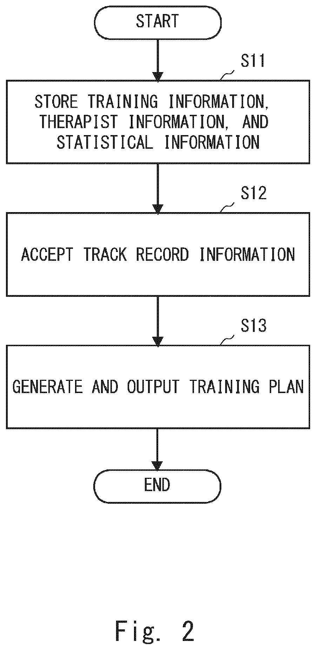

Referring next to Fig. 2, processing performed by the training system 10 will be described. Fig. 2 is a flowchart of the training system according to the first embodiment. First, the training system 10 stores the training information 111, the therapist information 112, and the statistical information 114 (Step S11). This processing is performed, for example, by the user supplying the aforementioned information to the training system 10 via the information input device 90.

Next, the track record information accepting unit 12 of the training system 10 accepts the track record information via the information input device 90 (Step S12). Then the plan generation unit 13 of the training system 10 generates a training plan and outputs the generated training plan to the display device 91 (Step S13).

The structure of the training system 10 and the processing performed by the training system 10 have been described above. With the above structure, the training system 10 is able to output the training plan in accordance with objective information.

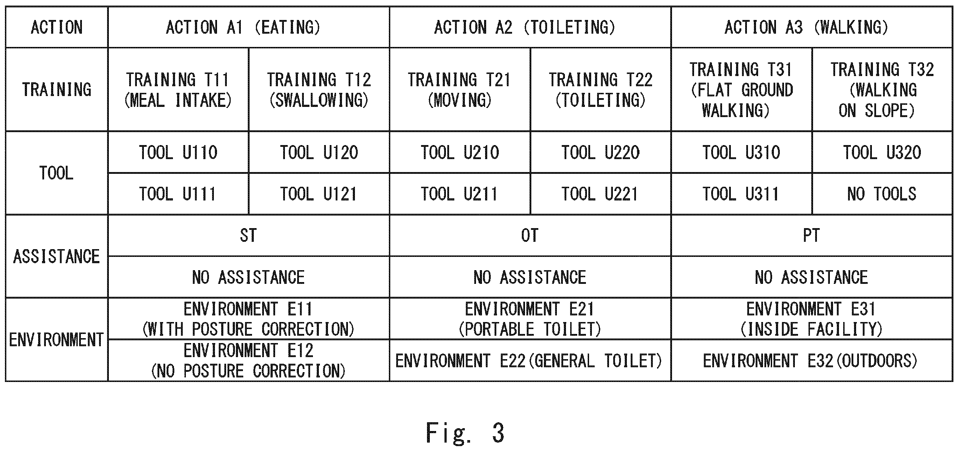

Referring next to Fig. 3, specific examples of the training information 111 and the therapist information 112 will be described. Fig. 3 is a diagram showing the training information and the therapist information according to the first embodiment. The table in Fig. 3 shows one example of information stored in the storage unit 11. Further, items listed in the table shown in Fig. 3 indicate possible combinations of training, tools, assistance, and environment that correspond to the respective physical actions. Specifically, the table shown in Fig. 3 at least includes the training information 111 indicating the content of training and the therapist information 112 indicating the type of the therapist.

The table shown in Fig. 3 shows, in the upper row, three physical actions including an action A1 (eating), an action A2 (toileting), and an action A3 (walking). Under the action A1, training T11 (meal intake) and training T12 (swallowing) are shown as training for recovering functions of the eating action. Under the training T11, tools used in the training T11 (tools U110 and U111) are shown. Further, under the training T12, tools used in the training T12 (tools U120 and U121) are shown. Further, under the column indicating tools, a therapist "ST" who assists the training T11 and the training T12 is shown as well as the description "no assistance". Under the column indicating assistance, an environment E11 (with posture correction) and an environment E12 (no posture correction) are shown. The environment E11 shows that posture correction is performed at the time of training. The environment E12 shows that the posture correction is not performed at the time of training.

The plan generation unit 13 is able to select the training T11 or the training T12 as training for the action A1. When the training T11 is performed, the plan generation unit 13 is able to select one or both of the tool U110 and the tool U111. When the training T12 is performed, the plan generation unit 13 is able to select one or both of the tool U120 and the tool U121. Likewise, when the training T11 or the training T12 is performed, the plan generation unit 13 is able to select whether or not the assistance should be provided by the ST. Further, when the training T11 or the training T12 is performed, the plan generation unit 13 is able to select the environment E11 or the environment E12.

Under the action A2, as training exercises for recovering functions of the toileting action, training T21 (moving) and training T22 (toileting) are shown. Under the training T21, tools used in the training T21 (tools U210 and U211) are shown. Further, under the training T22, tools used in the training T22 (tools U220 and U221) are shown. Further, under the column indicating tools, a therapist "OT" who assists the training T21 and the training T22 is shown as well as the description "no assistance". Further, under the column indicating assistance, an environment E21 (portable toilet) and an environment E22 (general toilet) are shown.

The plan generation unit 13 is able to select the training T21 or the training T22 as training for the action A2. Further, the plan generation unit 13 is able to select one or both of the tool U210 and the tool U211 when the training T21 is performed. The plan generation unit 13 is further able to select one or both of the tool U220 and the tool U221 when the training T22 is performed. Further, the plan generation unit 13 is able to select whether or not the assistance should be provided by the OT when the training T21 or the training T22 is performed. The plan generation unit 13 is able to select the environment E21 or the environment E22 when the training T21 or the training T22 is performed.

Under the action A3, training T31 (flat ground walking) and training T32 (walking on a slope) are shown as training for recovering functions of walking actions. Under the training T31, tools used in the training T31 (tools U310 and U311) are shown. Further, under the training T32, a tool used in the training T32 (tool U320) and "no tools" are shown. Further, under the column indicating tools, a therapist "PT" who assists the training T31 and the training T32 is shown as well as the description "no assistance". Further, under the column indicating assistance, an environment E31 (inside a facility) and an environment E32 (outdoors) are shown. Note that the aforementioned tools U310, U311, and U320 are, for example, a short leg brace, a long leg brace, a T-cane, and a four-leg stick.

The plan generation unit 13 is able to select the training T31 or the training T32 as training for the action A3. Further, the plan generation unit 13 is able to select one or both of the tool U310 and the tool U311 when the training T31 is performed. Further, the plan generation unit 13 is able to select the tool U320 or no tools when the training T32 is performed. Further, the plan generation unit 13 is able to select whether or not the assistance should be provided by the PT when the training T31 or the training T32 is performed. The plan generation unit 13 is able to select the environment E31 or the environment E32 when the training T31 or the training T32 is performed.

Specific examples of the training information 111 and the therapist information 112 have been described above. As described above, the storage unit 11 stores information including the training information 111 and the therapist information 112. The plan generation unit 13 selects the training for each action from combinations in the table shown in Fig. 3. The information shown in the table in Fig. 3 is merely one example for describing this embodiment and is not intended to limit the content of the training information 111 and the therapist information 112. For example, items regarding the actions are not limited to the actions A1 to A3. Further, the training, the tools, the assistance, and the environment in each action are not limited to the above content as well.

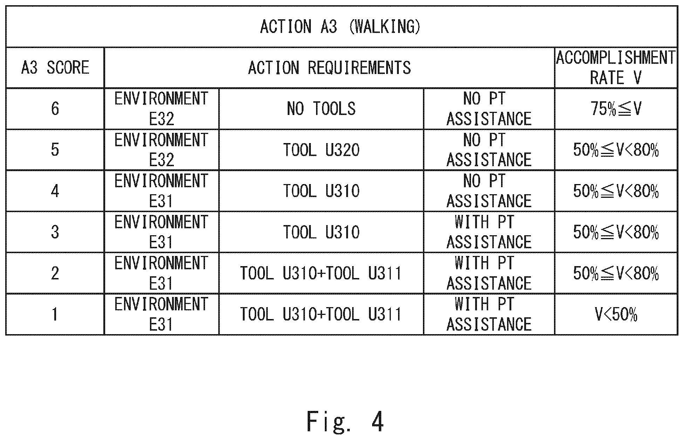

Referring next to Fig. 4, action levels will be described. Fig. 4 is a diagram showing an action level according to the first embodiment. The training system 10 uses the action level described below. The action level is an index indicating the level of the physical action set using at least information regarding the type of the tool that the trainee uses in the physical action and the level of achievement of the physical action.

The table shown in Fig. 4 shows, as one example of action levels in the physical actions, an action level related to the action A3 (walking). The left-side column in the table shown in Fig. 4 shows six stages from 1 to 6 as an "A3 score". Further, the table shown in Fig. 4 shows action requirements in a case in which the A3 score is 1 to 6, and the accomplishment rate V. The action requirements include the environment, and the presence or the absence of assistance by tools and the PT. The accomplishment rate V of the physical action can be determined, for example, from signals from a posture detection sensor which is worn by the trainee.

Assume a case, for example, the A3 score is determined to be 1. The trainee performs a predetermined action in the environment E31, using the tools U310 and U311, and with the assistance provided by the PT. In this case, when the achievement rate V of an action is smaller than 50 percent, the A3 score becomes 1. Further, as shown in the second row from the bottom in Fig. 4, when the achievement rate of the action is 50 percent or larger but smaller than 80 percent in the aforementioned action requirements, the A3 score becomes 2.

Further, as the action requirements, when a trainee performs a predetermined action in the environment E31, using the tool U310, and with the assistance of the PT and the achievement rate V of the action is 50 percent or larger but smaller than 80 percent, then the A3 score becomes 3. As the action requirements, when a trainee performs a predetermined action in the environment E3 1, using the tool U310, and without the assistance of the PT and the achievement rate V of the action is 50 percent or larger but smaller than 80 percent, then the A3 score becomes 4. Further, as the action requirements, when a trainee performs a predetermined action in the environment E32, using the tool U320, and without the assistance of the PT and the achievement rate V of the action is 50 percent or larger but smaller than 80 percent, then the A3 score becomes 5. As the action requirements, when a trainee performs a predetermined action in the environment E32, using the tool U320, and without the assistance of the PT and the achievement rate V of the action is 75 percent or larger, then the A3 score becomes 6.

One example of the action levels has been described above. The action level is composed of objective information, not based on subjective judgment. The training system 10 receives information regarding the action level of the trainee from the user by using this action level. Accordingly, the user is able to input track record information regarding physical actions of the trainee in a simple way. Further, the training system 10 is able to present the training plan to the user in an aspect in which the user can easily understand the training plan using the action level and can evaluate the training plan objectively.

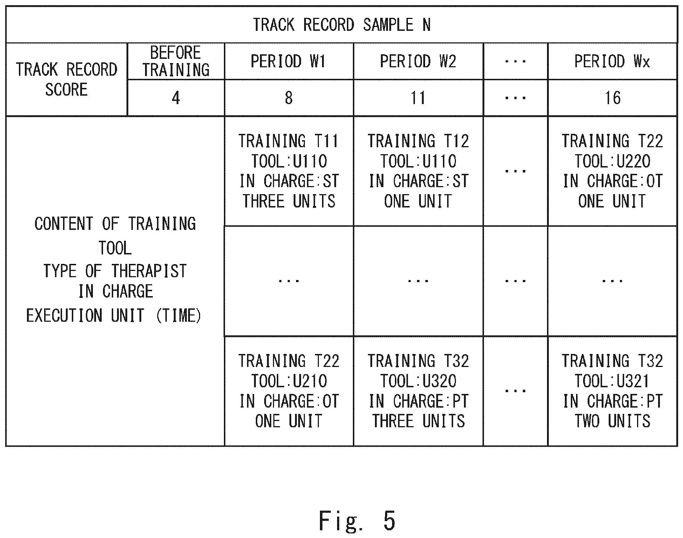

Referring next to Fig. 5, the statistical information will be described. Fig. 5 is a diagram showing the statistical information according to the first embodiment. The table in Fig. 5 shows an extraction of a track record sample N, which is the N-th sample included in the statistical information. The track record sample N is a piece of sample information that composes the statistical information. That is, the statistical information includes a plurality of track record samples as shown in the table in Fig. 5. The track record sample N includes information in which the content of the training that a predetermined trainee has performed, the type of the therapist who is in charge of the training, and the degree of recovery of the physical action of the trainee as a result of the training performed are associated with one another.

The table in Fig. 5 shows, in the upper row, that the track record sample is N. The table in Fig. 5 further shows, in the second row from the top, the track record score. The track record score is an action level which indicates the past records of the trainee in the track record sample N. In the track record score, the action level before the training and a transition of the action level from the first period W1 to the x-th period Wx are shown. The period W1 is, for example, a period in which one week is set as one unit. That is, the period W1 means the first week, the period W2 means the second week, and the period Wx means the x-th week. For the sake of convenience of explanation, the periods W3 to Wx-i and some columns indicating the details of the training are omitted.

In the row under the track record score, details of the training carried out in each period are shown. The details of the training include the content of the training, tools used in the training, the type of the therapist who is in charge, and the execution unit (time). The execution unit indicates a time period during which training is performed. For example, one unit is set as 30 minutes.

In the track record sample N shown in the table in Fig. 5, the track record score of the trainee before the training is 4. Further, the track record level after the period W1 is ended is 8. In the period W1, for example, training T11 and training T22 are performed. The table in Fig. 5 shows that the training T11 performed in the period W1 uses the tool U110, the therapist ST is in charge, and the three units (that is, 90 minutes) of training have been performed. The table in Fig. 5 further shows that the training T22 performed in the period W1 uses the tool U210, the therapist who is in charge of this training is OT, and the execution unit is one unit.

Likewise, the track record sample N shown in the table in Fig. 5 shows that the track record level after the period W2 is ended is 11. According to the table shown in Fig. 5, the training T12 and the training T32 are performed in the period W2. The tool used in the training T12 in the period W2 is the tool U110, the therapist who is in charge of this training is an ST, and the execution unit is one unit. The tool used in the training T32 in the period W2 is the tool U320, the therapist who is in charge of this training is a PT, and the execution unit is three units. Further, according to the table shown in Fig. 5, the training T22 and the training T32 are performed in the period Wx. The tool used in the training T22 in the period Wx is the tool U220, the therapist who is in charge of this training is an OT, and the execution unit is one unit. The tool used in the training T32 in the period Wx is the tool U321, the therapist who is in charge of this training is a PT, and the execution unit is two units.

As described above, the track record sample includes, for training performed by a predetermined trainee, tools that have been used, the therapist who is in charge, or a transition of the physical action level. It can also be said that the transition of the physical action level indicates the trainee's degree of recovery. The plan generation unit 13 generates the training plan using statistical information including a plurality of track record samples. Accordingly, the training system 10 is able to generate the training plan which enables the level of the trainee's physical action level to be efficiently improved.

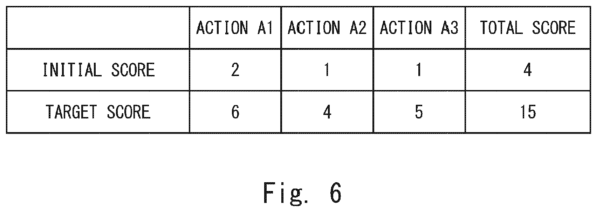

Referring next to Fig. 6, the track record information will be described. Fig. 6 is a diagram showing the track record information of the training system according to the first embodiment. The table shown in Fig. 6 shows initial scores and target scores of the action A1, the action A2, and the action A3. The right column in the table shown in Fig. 6 shows a total score obtained by adding the action scores of the action A1, the action A2, and the action A3 for each of the initial score and the target score.

The initial score is a score indicating the action level of the trainee just before the trainee starts training. The table shown in Fig. 6 shows that the initial score of the action A1 is 2, the initial score of the action A2 is 1, and the initial score of the action A3 is 1. The track record information accepting unit 12 accepts the above initial scores as the track record information of the trainee. The plan generation unit 13 generates the training plan from the accepted initial scores. The plan generation unit 13 is able to generate the training plan from the received initial scores, and the training information 111, the therapist information 112, and the statistical information 114 stored in the storage unit 11. However, as shown in the table shown in Fig. 6, the training system 10 may further accept the target of the training.

The target score is a target accomplished as a result of performing training according to the training plan generated by the training system 10. Just like the initial score, the target score is indicated by an action level. The table shown in Fig. 6 shows that the target score of the action A1 is 6, the target score of the action A2 is 4, and the target score of the action A3 is 5. When the training system 10 has accepted the target score as the track record information, the plan generation unit 13 generates the training plan so as to accomplish the accepted target.

In the training system 10, the track record information accepting unit 12 may further accept the profile information of the trainee. Accordingly, the training system 10 is able to generate the training plan in accordance with the trainee's profile.

Examples of the profile information will be described here. The profile information, which is information indicating information regarding a trainee, includes at least one of the following information (1)-(4).

### (1) Information indicating attributes of trainee

The information indicating attributes of the trainee includes, for example, the trainee's age, sex, physique (height, weight, etc.), and a score indicating the trainee's physical condition.

### (2) Symptom information of disease that trainee is suffering from

Symptom information may include a type(s) of a disease(s) (a name(s) of a disease(s) or a disorder(s)) that the subject has suffered from, such as a stroke (a cerebrovascular disorder) and a spinal cord injury. Further, the symptom information may also include, depending on the type of the disease, its classification. For example, strokes can be classified into cerebral infarction, intracranial hemorrhage (cerebral hemorrhage/subarachnoid hemorrhage), etc.

The symptom information may also include information indicating an initial symptom, a time when the symptom appears, and a current symptom in association with the above information. The symptom information may also include symptoms that are unlikely to be directly related to the rehabilitation in addition to the information indicating that the trainee needed to perform rehabilitation because of the symptoms included in the symptom information.

### (3) Trainee's assessment score based on stroke impairment assessment set

There is, for example, Stroke Impairment Assessment Set (SIAS) as an assessment method for quantifying an index for dysfunction caused by a stroke the trainee is suffering from. The SIAS may include a hip flexion test (Hip-Flex), a knee extension test (Knee-Ext), and a foot-pat test (Foot-Pat). Further, the SIAS may also include a lower limb tactile sensation (Touch L/E), a lower limb position sensation (Position L/E), an abdominal muscle strength (Abdominal), and a verticality test (Verticality).

### (4) Information indicating trainee's degree of recovery

The transition of the action level described above may be employed as the information indicating the trainee's degree of recovery. Specifically, when a certain amount of time has passed immediately after the trainee gets a disease such as a hemiplegia, changes in the action level during this period may be information indicating the trainee's degree of recovery. Further, the information indicating the trainee's degree of recovery may be the Brunnstrom Recovery Stage (Br.stage). The Brunnstrom Recovery Stage (Br.stage) is an indicator of recovery in which a recovery process of a hemiplegia is divided into six stages based on the observation. In this embodiment, the information indicating the trainee's degree of recovery may include, of the Br.stage, lower-limb items that are main items related to the walking training apparatus 100.

Although the profile information has been described in detail above, the profile information is not limited to the above-described items and may be information of types different from those described above as long as it includes information satisfying the purpose of the profile information. The profile information may also include additional information such as a date and a time when information is measured.

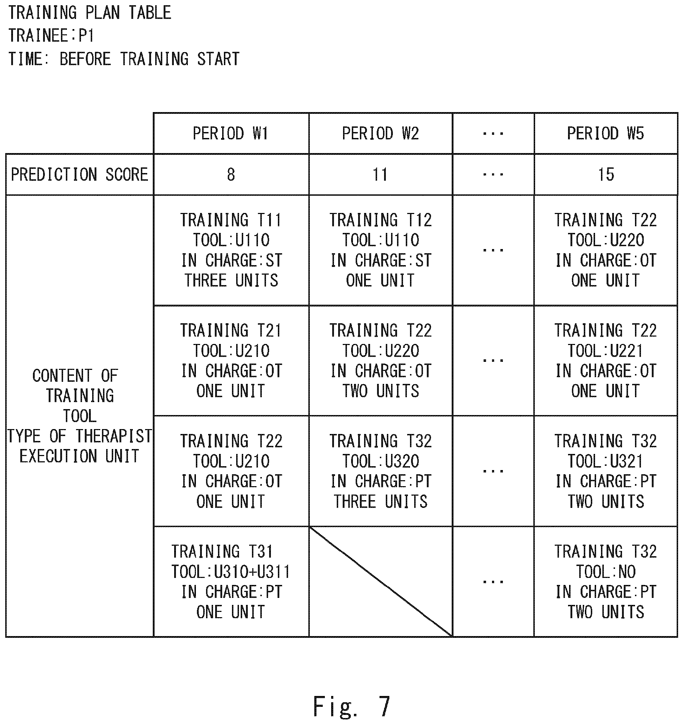

Referring next to Fig. 7, the training plan generated by the plan generation unit 13 will be described. Fig. 7 is a first diagram showing the training plan according to the first embodiment. Fig. 7 shows the training plan generated by the plan generation unit 13 that has accepted the track record information shown in Fig. 6. Further, the configuration of the table shown in Fig. 7 is similar to the sample information shown in Fig. 5. The training plan shown in the table in Fig. 7 is the one of the trainee P1, and the time is before the start of the training.

In the table shown in Fig. 7, the upper row shows periods W1 to W5. Under the periods W1 to W5, prediction scores are shown. The prediction scores are the predicted action levels of the trainee after the training in periods indicated above the column indicating the respective numerical values is ended. The table in Fig. 7 shows that the action level after the period W1 is ended is 8, the action level after the period W2 is ended is 11, and the action level after the period W5 is ended is 15. For the sake of convenience of explanation, the periods W3 and W4 are omitted in the table in Fig. 7.

Under the column of the prediction scores, details of the training planned in each period are shown. Specifically, training exercises planned in the period W1 are the training T11, the training T21, the training T22, and the training T31. The training T11 uses the tool U110, the therapist ST is in charge, and the execution unit is three units. The training T21 uses the tool U210, the therapist OT is in charge, and the execution unit is one unit. The training T22 uses the tool U210, the therapist OT is in charge, and the execution unit is one unit. The training T31 uses the tools U310 and U311, the therapist PT is in charge, and the execution unit is one unit.

The training exercises planned in the period W2 are training T12, training T22, and training T32. The training T12 uses the tool U110, the therapist ST is in charge, and the execution unit is one unit. The training T22 uses the tool U220, the therapist OT is in charge, and the execution unit is two units. The training T32 uses the tool U320, the therapist PT is in charge, and the execution unit is three units.

The training exercises planned in the period W5 are training T22, training T22, training T32, and training T32. One training T22 uses the tool U220, the therapist OT is in charge, and the execution unit is one unit. The other training T22 uses the tool U221, the therapist OT is in charge, and the execution unit is one unit. One training T32 uses the tool U321, the therapist PT is in charge, and the execution unit is two units. The other training T32 does not use any tools, the therapist PT is in charge, and the execution unit is two units.

As described above, the plan generation unit 13 generates the content of the training for each period, the tool to be used, the type of the therapist who is in charge, and the execution unit, which is a time when the training is executed. The training plan shown in the table in Fig. 7 is generated by the plan generation unit 13 combining the training information shown in Fig. 3 with the therapist information. Further, the plan generation unit 13 generates the above training plan in view of the action level shown in Fig. 4, the statistical information shown in Fig. 5, and the track record information shown in Fig. 6.

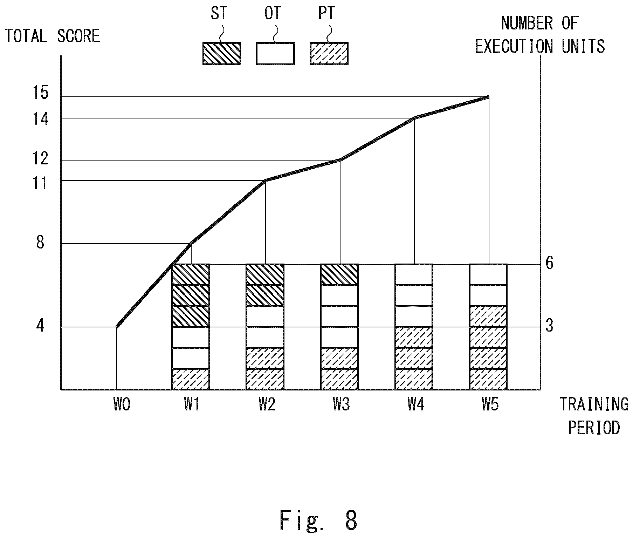

Fig. 8 is a second diagram showing the training plan according to the first embodiment. Fig. 8 shows the training plan shown in Fig. 7 in a display form different from that of Fig. 7. The line graph in Fig. 8 shows a transition of the action level for each training period. The horizontal axis of the graph shows the training period. The training period W0 means a period before training is started and the training W1 to training W5 each indicates a timing after each training period is ended. The vertical axis on the left side of the graph, which corresponds to the line graph, indicates the total score of the action levels. As shown in the line graph in Fig. 8, the training is planned so that the trainee advances the training from the initial score 4 to the target score 15.

The bar graph in Fig. 8 shows a relation between the types of therapists for each training period and the number of execution units that each of the therapists is in charge of. The vertical axis on the right side of the graph, which corresponds to the bar graph, indicates the number of execution units. The execution unit of the training performed in each period is six units. However, since the content of the training exercises in the six units varies from one another, the execution units of the training exercises that the respective therapists are in charge of are different from one another. In the period W1, for example, the ST is in charge of three units of training, the OT is in charge of two units of training, and the PT is in charge of one unit of training. In the period W2, the ST is in charge of two units of training, the OT is in charge of two units of training, and the PT is in charge of two units of training. In this manner, the plan generation unit 13 is able to present the training in each training period and the type of the therapist who is in charge of this training.

The first embodiment has been described above. The training plan generated by the training system 10 according to the first embodiment is not limited to the aforementioned one related to the physical actions. The physical actions regarding which the training plan is generated include various other actions related to ADL. Further, while the PT, the OT, and the ST have been mentioned as the therapists in the above-described description, a person who is in charge of training or a person who assists the trainee in the physical actions may include, for example, a nurse in a medical institute or a family member of the trainee. The number of training exercises related to the physical actions according to this embodiment is not limited as long as it is plural.

In the training system 10, the track record information accepting unit 12 may accept information from a sensor that has detected physical actions of the trainee as the track record information. In this case, the training system 10 includes, for example, a communication function, and is connected to a predetermined sensor apparatus in such a way that they can communicate with each other. Then the trainee wears a sensor that is able to detect the posture of the trainee and the state of the physical action. According to the above structure, the training system 10 is able to acquire information regarding the physical actions of the trainee. Accordingly, the training system 10 is able to determine, for example, whether or not the trainee has fallen during walking. As described above, the above sensor may be a contact-type sensor worn and used by the trainee or may be a non-contact sensor used by the trainee without wearing it. The contact-type sensor is, for example, a pressure sensor, an acceleration sensor, an angle sensor etc. Further, the non-contact sensor is, for example, an image sensor, an infrared sensor, a distance measurement sensor etc.

Further, in the training system 10, the track record information accepting unit 12 may accept image information from a camera that has captured physical actions of a trainee as the track record information. In this case, the training system 10 is able to recognize, from the accepted image information, the state of the physical actions performed by the trainee and thereby determine the levels of achievement of these physical actions. Accordingly, the training system 10 is able to easily acquire the track record information.

As described above, the training system 10 is able to output the training plan based on the objective information and the objective track record information. Therefore, according to the first embodiment, it is possible to provide a training system and the like that efficiently perform function recovery training.

### <Second Embodiment>

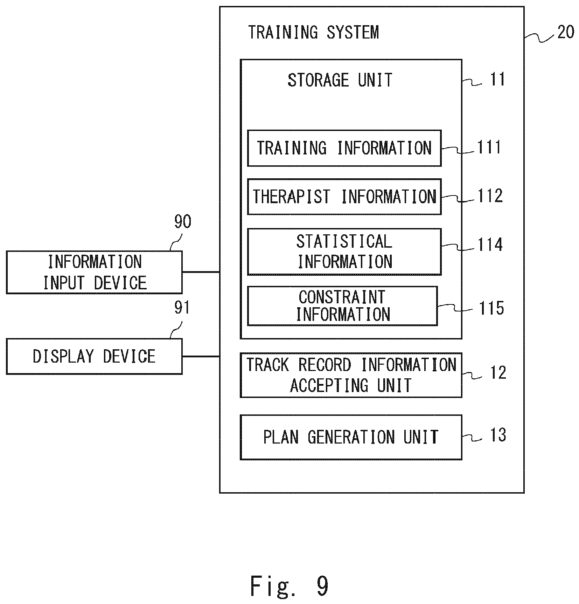

Next, a second embodiment will be described. The second embodiment is different from the first embodiment in that a training plan is generated in view of predetermined constraint conditions. Fig. 9 is a block diagram of a training system according to the second embodiment. A training system 20 shown in Fig. 9 includes constraint information 115 in the storage unit 11.

The constraint information is information regarding constraint conditions regarding the time of the training that a trainee or a therapist performs in a predetermined period. The constraint information includes, for example, limitation on the number of execution units of the training exercise that the trainee carries out in a day. Alternatively, the constraint information includes limitation on the number of execution units of the training exercises that a therapist is in charge in one day. The constraint information may be stored in the storage unit 11 in advance or may be rewritable information.

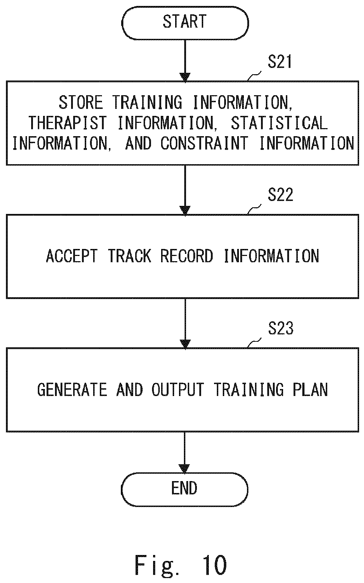

Referring next to Fig. 10, processing performed by the training system 20 will be described. Fig. 10 is a flowchart of the training system according to the second embodiment. First, the training system 20 stores the training information 111, the therapist information 112, the statistical information 114, and the constraint information 115 (Step S21). This processing is performed, for example, by the user supplying the aforementioned information to the training system 10 via the information input device 90.

Next, the track record information accepting unit 12 of the training system 20 accepts the track record information via the information input device 90 (Step S22). Then the plan generation unit 13 of the training system 20 generates a training plan and outputs the generated training plan to the display device 91 (Step S23).

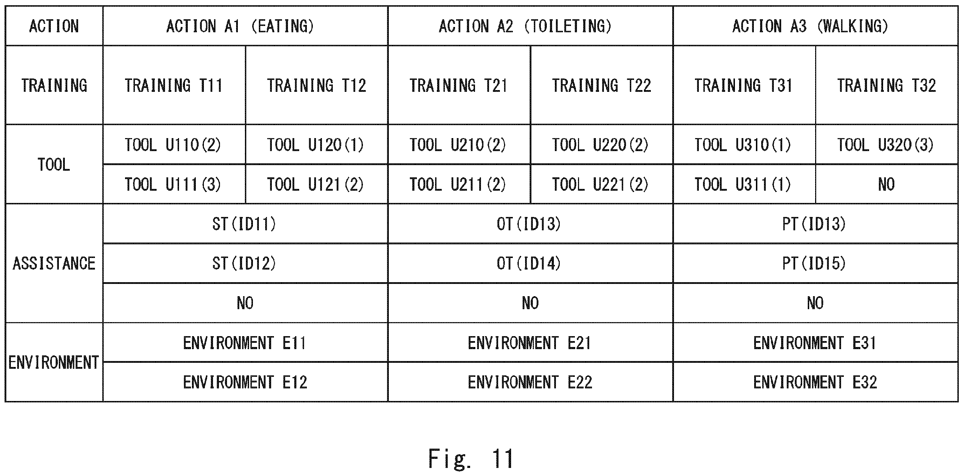

Referring next to Fig. 11, an example of the training information according to the second embodiment will be described. Fig. 11 is a diagram showing the training information according to the second embodiment. The training information shown in the table in Fig. 11 is different from the training information according to the first embodiment in that the training information shown in the table in Fig. 11 includes information regarding the number of tools stored in a training facility. Further, the training information shown in the table in Fig. 11 is different from the training information according to the first embodiment in that the training information shown in the table in Fig. 11 includes identification information on a therapist in addition to the type of the therapist. In the following, the table shown in Fig. 11 will be described mainly with regard to the difference between the training information according to this embodiment and the training information according to the first embodiment.

In the table shown in Fig. 11, the column indicating tools shows, besides information regarding the types of the tools, the number of tools stored in the training facility by the numbers in parentheses. For example, the tools that can be used in the training T11 are tools U110 and U111, and there are two tools U110 and three tools U111. Likewise, each of the number of tools U210, the number of tools U211, and the number of tools U221 shown in the table in Fig. 11 is two. Further, there are one tool U310 and one tool U311. Furthermore, there are three tools U320.

In the table shown in Fig. 11, the column indicating the therapists shows, besides information regarding the types of the therapists, identification information on the therapists by identifiers in parentheses. One person has one identifier. For example, the therapists who can be in charge of the training T11 and the training T12 are STs: a person who has an identifier ID11 and a person who has an identifier ID12. Likewise, the therapists who can be in charge of the training T21 and the training T22 are OTs: a person who has an identifier ID13 and a person who has an identifier ID14. The therapists who can be in charge of the training T31 and the training T32 are PTs: a person who has an identifier ID13 and a person who has an identifier ID15.

The identifier ID13 is shown in the columns OT and PT. That is, the person who has the identifier ID13 can be in charge of the training T21 and the training T22 as an OT and can also be in charge of the training T31 and the training T32 as a PT.

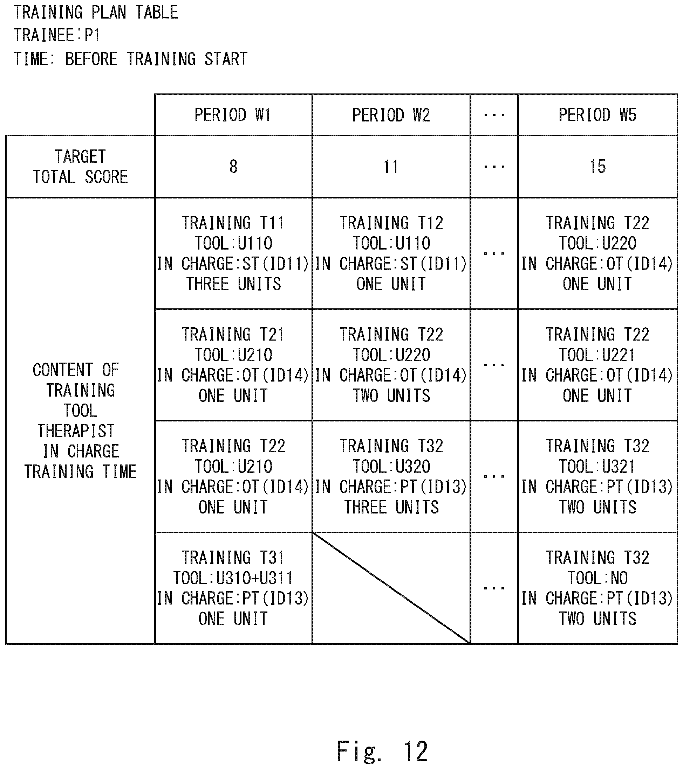

Referring next to Fig. 12, the training plan generated by the training system 20 according to the second embodiment will be described. Fig. 12 is a first diagram showing the training plan according to the second embodiment. The training plan shown in Fig. 12 is different from the training plan according to the first embodiment in that identification information is included in the information on a therapist who is in charge of training.

In the training plan shown in the table in Fig. 12, in the columns of the respective training exercises, identification information on the therapists are included besides the types of the therapists who are in charge of the training. For example, the ST who is in charge of the training T11 in the period W1 is the person who has the identifier ID11. Likewise, the OT who is in charge of the training T21 and the training T22 in the period W1 is the person who has the identifier ID14. Further, the PT who is in charge of the training T31 in the period W1 is the person who has the identifier ID13. In each of the training exercises in the following periods W2-W5 as well, identification information on the therapists is included.

When the plan generation unit 13 generates the training plan after a predetermined timing, the plan generation unit 13 aggregates combinations of the trainee PI, the training that the trainee P1 has performed before the predetermined timing, and identification information on the therapist who is in charge of the training, thereby generating the training plan in accordance with the length of the execution period of the training determined in accordance with a combination of the trainee P1 with the identification information on the therapist.

The plan generation unit 13 selects, for example, the PT who is in charge of the training T32 in the training plan in the period W5. In this case, the plan generation unit 13 compares a period in which the PT having the identifier ID13 has been in charge of training of the trainee P1 with a period in which the PT having the identifier ID15 has been in charge of training of the trainee P1. The period in which the PT having the identifier ID13 has been in charge of training of the trainee P1 is referred to as a first period and a period in which the PT having the identifier ID15 has been in charge of training of the trainee P1 is referred to as a second period shorter than the first period. At this time, the plan generation unit 13 sets the priority of the PT having the identifier ID13 who takes care of the trainee P1 for the first period to be higher than the priority of the PT of the identifier ID15 who takes care of the trainee P1 for the second period. Then the plan generation unit 13 generates a training plan based on the set priority. As a result, the plan generation unit 13 selects the PT having the identifier ID13 as a therapist who is in charge of the training T32 in the period W5.

According to the above configuration, the training system 20 is able to generate a training plan in which a combination of a specific trainee with a specific therapist is taken into account. Therefore, the training system 20 is able to generate a training plan in which a specific therapist preferentially assists a specific trainee.

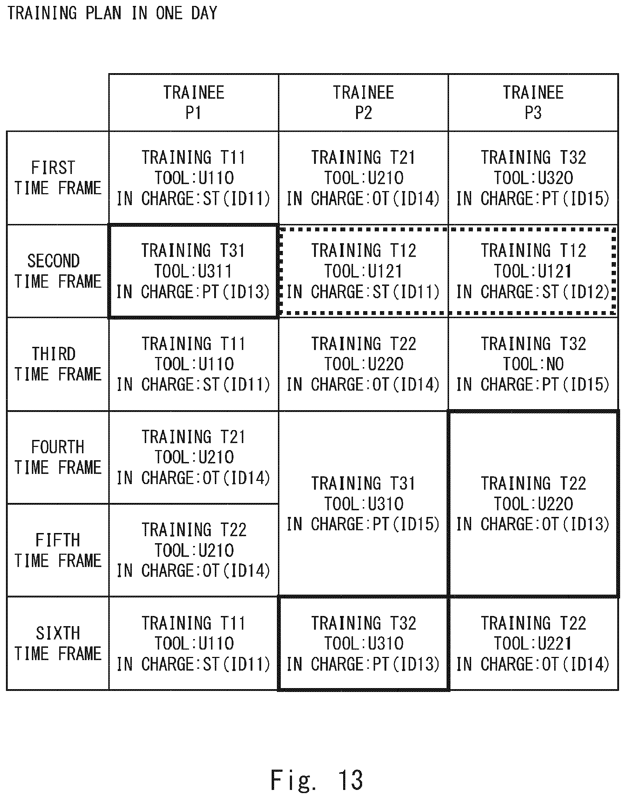

Referring next to Fig. 13, the training plan generated by the plan generation unit 13 will be further described. Fig. 13 is a second diagram showing the training plan according to the second embodiment. The table shown in Fig. 13 shows the plan of the training exercises that the trainees PI, P2, and P3 respectively perform. In Fig. 13, the training plan indicating training exercises the respective trainees perform in one day is shown in the order in which they are performed. In the table shown in Fig. 13, a "first time frame" to a "sixth time frame" are shown vertically in order in the left column, the "trainee P1'', the "trainee P2", and the "trainee P3" are shown horizontally in the upper row, and details of the training exercises that the respective trainees perform are shown in a matrix form.

The "first time frame" is a period of the training assigned to the earliest time of a day. The "second time frame" is a period of the training assigned after the first time frame. Likewise, the third to sixth time frames are assigned as periods of the training. Each time frame is a constant period such as, for example, 30 minutes. In this manner, the plan generation unit 13 generates the training plan including an order in which a plurality of training exercises are offered to one trainee or time at which a plurality of training exercises are started. The plan generation unit 13 further generates the training plan to be performed in parallel for a plurality of trainees.

In the table shown in Fig. 13, the trainee P1 performs, for example, training T11 in the first time frame. In this training, the tool U110 is used and the therapist who is in charge of this training is an ST having the identifier ID11. Likewise, the trainee P1 performs the training T31 in the second time frame. In this training, the tool U311 is used and the therapist who is in charge of this training is a PT having the identifier ID13. The same is applied to the subsequent training exercises. The table shows that the trainee P1 performs, in the third time frame, the training T11, using the tool U110, and the ST (ID11) is in charge of this training.

When the plan generation unit 13 generates the training plan for a plurality of trainees as shown in Fig. 13, the plan generation unit 13 generates the training plan by restricting the number of training exercises that a therapist having one identification information is in charge during the same time. Specifically, the plan generation unit 13 generates the training plan in such a way that the same identification information do not overlap at the same time. For example, the person who has the identifier ID13 is in charge of the training T31 of the trainee P1 as a PT in the second time frame. Further, the person who has the identifier ID13 is in charge of the training T22 of the trainee P3 as an OT in the fourth and fifth time frames. Further, the person who has the identifier ID13 is in charge of the training T32 of the trainee P2 as a PT in the sixth time frame.

When the training plan for the plurality of trainees as shown in Fig. 13 is generated, the plan generation unit 13 generates the training plan in such a way that the number of tools used during the same time does not exceed the number of tools stored in the facility in a case in which tools of the same kind are used during the same time. In the second time frame, for example, the trainees P2 and P3 both perform the training T12 using the tool U121. Fig. 11 shows that the number of tools 121 that are stored in the facility is two. Therefore, as shown in Fig. 13, two training exercises that use the tool U121 can be concurrently performed.

While the second embodiment has been described above, the configuration of the training system 20 according to the second embodiment is not limited to the aforementioned configuration. The training system 20 may show the training plan for each type of the therapist instead of showing it for each of the trainees as shown in Fig. 13. Alternatively, the training system 20 may show the training plan for each of the identification information pieces. According to the aforementioned structure, the training system 20 is able to generate a training plan that can be easily understood by the therapist.

The training system 20 may further accept, when it accepts the track record information, preset constraint information in addition to the track record information. Note that the target described in the first embodiment may also be included in the constraint information.

Further, the plan generation unit 13 may generate a plurality of different training plans that can be executed and output the generated plans. Accordingly, the training system is able to present a plurality of training plans that can be selected to the user.

As described above, the training system 20 outputs the training plan based on the objective information and the objective track record information. Further, the training system 20 generates the training plan in a flexible way in accordance with various constraint conditions. Therefore, according to the second embodiment, it is possible to provide a training system and the like that enable a trainee to perform function recovery training efficiently.

Note that the present disclosure is not limited to the above embodiments and may be changed as appropriate without departing from the spirit of the present disclosure.

The program described above can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A training system (10) configured to generate a training plan for enabling a trainee to perform a plurality of kinds of training for the purpose of recovering functions of a physical action of the trainee, the training system comprising:
a storage unit (11) configured to store training information including the content of the plurality of kinds of training, therapist information indicating the type of a therapist who is in charge of the training and assists the trainee, and statistical information regarding the function recovery in the training performed by a plurality of other trainees;
a track record information accepting unit (12) configured to accept track record information regarding the physical action of the trainee; and
a plan generation unit (13) configured to generate, when it has accepted the track record information, a training plan including the content of the training that the trainee conducts, the type of the therapist, and an execution period of the training based on the training information, the therapist information, and the statistical information and outputs the generated training plan.

2. The training system according (10) to Claim 1, wherein the storage unit (11) stores, as the statistical information, information in which the content of the training performed by the plurality of other trainees, types of the therapists who have been in charge of the training, and degrees of recovery of physical actions of the plurality of other trainees are associated with one another.

3. The training system (10) according to Claim 1 or 2, wherein the track record information accepting unit further accepts profile information of the trainee.

4. The training system (10) according to any one of Claims 1 to 3, wherein
the track record information accepting unit (12) accepts information regarding an action level of the trainee set using at least information regarding the type of a tool that the trainee uses in the physical action and the level of achievement of the physical action, and
the plan generation unit (13) generates the training plan based on the action level.

5. The training system (10) according to any one of Claims 1 to 4, wherein
the track record information accepting unit (12) further accepts a target of the training, and
the plan generation unit (13) generates the training plan based on the target.

6. The training system (10) according to any one of Claims 1 to 5, wherein
the storage unit (11) further stores identification information on the therapist as the therapist information, and
the plan generation unit (13) generates, when it generates the training plan after a predetermined timing, the training plan in accordance with the length of an execution period of the training determined in accordance with a combination of the trainee with the therapist based on a combination of the trainee, the training that the trainee has performed before the predetermined timing, and the identification information on the therapist who has been in charge.

7. The training system (10) according to Claim 6, wherein the plan generation unit (13) sets, in the combination, the priority of a first therapist who takes care of the trainee for a first period to be higher than the priority of a second therapist who takes care of the trainee for a second period shorter than the first period, and generates the training plan based on the set priority.

8. The training system (10) according to Claim 6, wherein the plan generation unit (13) generates, when it generates the training plan for a plurality of trainees, the training plan by restricting the number of training exercises that the therapist having one identifier is in charge during the same time based on the identification information.

9. The training system (10) according to any one of Claims 1 to 8, wherein
the storage unit (11) further stores constraint information regarding constraint conditions regarding a time of the training that the trainee or the therapist performs in a preset period, and
the plan generation unit (13) generates the training plan based on the constraint conditions.

10. The training system (10) according to any one of Claims 1 to 9, wherein the plan generation unit (13) generates, when it generates the training plan including a plurality of training exercises for one trainee, the training plan including an order in which the plurality of training exercises are performed or time when the plurality of training exercises are started.

11. The training system according to any one of Claims 1 to 10, wherein the plan generation unit generates a plurality of different training plans that can be executed and outputs the generated plans.

12. The training system (10) according to any one of Claims 1 to 11, wherein the track record information accepting unit (12) accepts information from a sensor that has detected the physical action of the trainee as the track record information.

13. The training system (10) according to any one of Claims 1 to 11, wherein the track record information accepting unit (12) accepts image information from a camera that has captured images of the physical action of the trainee as the track record information.

14. A training method for generating a training plan for enabling a trainee to perform a plurality of kinds of training for the purpose of recovering functions of a physical action of the trainee, the training method comprising:
a storing step for storing training information including the content of the plurality of kinds of training, therapist information indicating the type of a therapist who is in charge of the training and assists the trainee, and statistical information regarding the function recovery in the training performed by a plurality of other trainees;
a track record information accepting step for accepting track record information regarding the physical action of the trainee; and
a plan generation step for generating, when the track record information has been accepted, a training plan including the content of the training that the trainee conducts, the type of the therapist, and an execution period of the training based on the training information, the therapist information, and the statistical information and outputs the generated training plan.

15. A program for causing a computer to execute a training method for generating a training plan for enabling a trainee to perform a plurality of kinds of training for the purpose of recovering functions of a physical action of the trainee, the training method comprising:
a storing step for storing training information including the content of the plurality of kinds of training, therapist information indicating the type of a therapist who is in charge of the training and assists the trainee, and statistical information regarding the function recovery in the training performed by a plurality of other trainees;
a track record information accepting step for accepting track record information regarding the physical action of the trainee; and
a plan generation step for generating, when the track record information has been accepted, a training plan including the content of the training that the trainee conducts, the type of the therapist, and an execution period of the training based on the training information, the therapist information, and the statistical information and outputs the generated training plan.
